# FASCICULE DE BREVET EUROPEEN

(11) **EP 3 814 231 B1**
(45) Date de publication et mention de la délivrance du brevet: **18.09.2024**
(21) Numéro de dépôt: 19790590.4
(22) Date de dépôt: 07.06.2019
(51) Int. Cl.: B64F 5/40, F01M 11/04, F16N 31/00, B64C 1/14, B60S 5/00, F01D 25/32

(54) **DISPOSITIF ET PROCÉDÉ DE VIDANGE ET SURVEILLANCE DE FLUIDE DRAINÉ D'UN MOTEUR D'AÉRONEF**
VORRICHTUNG UND VERFAHREN ZUR ENTLEERUNG UND ÜBERWACHUNG EINES AUS EINEM FLUGZEUGMOTOR ABGELASSENEN FLUIDS
DEVICE AND METHOD FOR EMPTYING AND MONITORING OF A FLUID DRAINED FROM AN AIRCRAFT ENGINE

(30) Priorité: 14.06.2018 FR 1855222
(43) Date de publication de la demande: 05.05.2021
(73) Titulaire: SAFRAN AIRCRAFT ENGINES, 75015 Paris (FR)
(72) Inventeur: GARNIER, Alméric Pierre Louis, 77550 Moissy-Cramayel (FR); COUPARD, Josselin Xavier, 77550 Moissy-Cramayel (FR)
(74) Mandataire: Cabinet Beau de Loménie
(86) Numéro de dépôt international: PCT/FR2019/051392
(87) Numéro de publication internationale: WO 2019/239047

(56) Documents cités:
- WO-A1-93/15309
- US-A- 5 452 695
- US-A- 5 610 341
- US-A- 5 893 398
- US-A1- 2002 148 788
- US-A1- 2004 060 344
- US-A1- 2004 165 185
- US-A1- 2012 210 769
- US-A1- 2015 343 346
- US-A1- 2017 089 236
- US-B1- 6 860 300

## Description

### Arrière-plan de l'invention

La présente divulgation concerne le domaine de la propulsion aéronautique, et plus particulièrement un dispositif de vidange et surveillance pour vidanger et surveiller un fluide drainé d'un moteur d'aéronef.

Dans le présent contexte, on entend par « moteur d'aéronef » tout propulseur embarqué à bord d'un aéronef et destiné à sa propulsion en vol, en particulier les moteurs à turbine à gaz, comme par exemple les turboréacteurs à simple ou double flux, les turbopropulseurs ou les turbomoteurs, mais aussi les moteurs à pistons ou électriques.

Normalement les moteurs d'aéronef comprennent des pièces mobiles soumises à des contraintes mécaniques et thermiques élevées. Il est donc généralement important d'alimenter en fluide lubrifiant certains éléments du moteur, notamment les paliers de support d'arbres rotatifs. En outre, les moteurs d'aviation peuvent comprendre aussi des actionneurs hydrauliques, qui peuvent notamment utiliser du carburant comme fluide hydraulique et/ou comme lubrifiant. Une partie de ces fluides peut s'échapper par vaporisation ou écoulement liquide, et représente une source potentielle de pollution environnementale. Ainsi, pour réduire ou éviter cette pollution, des réservoirs embarqués ont été proposés, notamment dans la publication de demande internationale de brevet WO 2015/082833 A1, pour recevoir des fluides drainés du moteur, et les contenir jusqu'à leur vidange contrôlée.

En outre, afin de surveiller l'état d'un moteur, et notamment de son fluide lubrifiant, il est connu de procéder à des analyses de ce fluide lubrifiant. Toutefois, ceci exige normalement le prélèvement d'échantillons de fluide lubrifiant du moteur, et leur transport jusqu'à un dispositif d'analyse, ce qui peut être difficile lorsque ce moteur est embarqué à bord d'un aéronef.

Finalement, il est aussi connu d'intégrer des capteurs de qualité de fluide lubrifiant directement dans le circuit de lubrification d'un aéronef. Toutefois, cette intégration peut être difficile à cause de la nécessité de maintenir la circulation du fluide lubrifiant à travers ce circuit.

Des dispositifs de vidange et surveillance pour vidanger et surveiller des fluides moteurs ont notamment été divulgués dans les documents US5452695, WO9315309 et US2017089236. Document US5452695 divulgue un procédé et dispositif pour changer l'huile et mesurer la consommation d'huile dans un moteur à combustion interne, ayant un système de passage de distribution de lubrification d'huile interne avec un filtre à huile et un réservoir d'huile.

### Objet et résumé de l'invention

La présente divulgation vise à remédier à ces inconvénients, en proposant un dispositif de vidange et surveillance pour vidanger et surveiller un fluide drainé d'un moteur d'aéronef, qui permette une analyse plus immédiate de ce fluide afin de contribuer à la surveillance du moteur.

Suivant un premier aspect, ce but peut être atteint grâce à ce que le dispositif de vidange et surveillance comprend une perche articulée incorporant un premier passage d'admission extensible apte à établir une connexion hermétique avec un passage de vidange disposé sur l'aéronef pour recevoir le fluide drainé du moteur directement depuis l'aéronef, et à ce que le dispositif de vidange et surveillance comprend aussi un ensemble capteur de qualité pour capter au moins un paramètre de qualité du fluide drainé du moteur admis à travers le premier passage d'admission. Cet ensemble capteur de qualité peut notamment comprendre un capteur de viscosité pour capter une viscosité du fluide drainé du moteur et/ou un ensemble capteur de polluants pour capter une présence de polluants dans le fluide drainé du moteur. Plus particulièrement, cet ensemble capteur de polluants peut comprendre un capteur de conductivité électrique, un capteur de particules ferromagnétiques, un capteur optique et/ou un capteur acoustique. Le capteur de conductivité électrique peut notamment être configuré pour contribuer à la détection d'eau et/ou d'autres polluants dans le fluide drainé du moteur, tandis que le capteur optique ou acoustique peut être configuré pour effectuer une mesure de transmission, absorption, réflexion et/ou réfraction du fluide contenu dans le réservoir embarqué, sur une ou plusieurs longueurs d'onde, notamment afin d'en caractériser la composition, décantation et/ou stratification du fluide drainé du moteur.

Grâce à la réception directe depuis l'aéronef du fluide drainé du moteur et à l'installation de l'ensemble capteur de qualité dans le dispositif de vidange et surveillance, il est ainsi possible de contribuer immédiatement à une surveillance de l'état du moteur lors de la vidange de ce fluide, qui peut notamment avoir été reçu et stocké dans un réservoir embarqué avant sa vidange, et notamment mais pas seulement à une surveillance de l'état de son fluide lubrifiant, à travers au moins de la qualité du fluide drainé naturellement du moteur, et cela donc sans devoir interférer dans la circulation normale des fluides de fonctionnement au sein du moteur. En outre, le fluide analysé n'étant pas nécessairement filtré, il peut être plus riche en informations. Il est aussi possible de minimiser ou éliminer toute pollution externe lors de la vidange du fluide drainé vers le dispositif de vidange et surveillance.

En outre, le dispositif de vidange et surveillance peut comporter en outre un premier réservoir, relié au premier passage d'admission, avec un premier capteur de niveau pour capter un niveau de fluide dans le premier réservoir. Ainsi, il est possible de surveiller, non seulement la qualité du fluide drainé, mais aussi sa quantité, permettant ainsi l'identification d'éventuels blocages et/ou fuites dans sa circulation au sein du moteur.

Le dispositif de vidange et surveillance peut comprendre en outre, en aval du premier ensemble capteur de qualité, au moins un appareil de traitement, pour le traitement du fluide drainé du moteur ayant été admis à travers le premier passage d'admission. Dans ce contexte, le terme « en aval » doit être compris par rapport à un sens de circulation, à travers le dispositif de vidange et surveillance, du fluide drainé du moteur et ayant été admis à travers le premier passage d'admission. L'au moins un appareil de traitement peut comprendre, par exemple, une centrifugeuse et/ou une cuve de décantation pour permettre une séparation au moins partielle des composants du fluide drainé du moteur et assurer ainsi son traitement écoresponsable, par exemple en facilitant son recyclage.

Le dispositif de vidange et surveillance peut aussi comprendre au moins un deuxième passage d'admission pour recevoir, directement depuis l'aéronef, du fluide drainé du moteur, et un deuxième ensemble capteur de qualité pour capter au moins un paramètre de qualité du fluide drainé du moteur ayant été admis à travers le deuxième passage d'admission. Il peut ainsi être possible de capter séparément des paramètres de qualité de fluide drainé de régions différentes du moteur, facilitant ainsi une surveillance et un diagnostic plus précis de celui-ci.

La perche du dispositif de vidange et surveillance peut être aussi articulée et télescopique.

Un deuxième aspect de cette divulgation concerne un véhicule aéroportuaire comprenant le dispositif de vidange et surveillance susmentionné. En outre, ce véhicule aéroportuaire peut comprendre un dispositif de pilotage autonome. Ainsi, le véhicule aéroportuaire peut déplacer le dispositif de vidange et surveillance vers l'aéronef à l'arrêt ou même en déplacement sur les pistes d'un aéroport pour effectuer la vidange et une analyse immédiate du fluide drainé du moteur.

Alternativement, toutefois, suivant un troisième aspect, cette divulgation concerne aussi une installation aéroportuaire fixe comprenant le dispositif de vidange et surveillance susmentionné.

Un quatrième aspect de cette divulgation concerne un procédé de vidange et surveillance pour vidanger et surveiller un fluide drainé d'un moteur d'aéronef, le procédé de vidange et surveillance pouvant comprendre une étape d'admission, par un premier passage d'admission extensible incorporé dans une perche articulée d'un dispositif de vidange et surveillance, du fluide drainé du moteur d'aéronef, directement depuis l'aéronef, et une étape de capture, à travers un ensemble capteur de qualité, d'au moins un paramètre de qualité du fluide drainé du moteur d'aéronef ayant été admis à travers le premier passage d'admission. Ce procédé peut comprendre en outre une étape, préalable, de déplacement du dispositif de vidange et surveillance, à bord d'un véhicule aéroportuaire, vers l'aéronef, et en particulier un pilotage autonome du véhicule pendant cette étape de déplacement.

Par ailleurs, ce procédé comprend en outre une étape de connexion hermétique du premier passage d'admission avec un passage de vidange sur l'aéronef, pouvant notamment être effectuée de manière automatique.

Alternativement, toutefois, le procédé peut comprendre une étape de vidange par gravité du fluide drainé d'un moteur d'aéronef, directement de l'aéronef vers une cuvette de réception du passage d'admission.

La perche du dispositif de vidange et surveillance peut être aussi articulée et télescopique.

### Brève description des dessins

L'invention sera bien comprise et ses avantages apparaîtront mieux, à la lecture de la description détaillée qui suit, de modes de réalisation représentés à titre d'exemples non limitatifs, l'invention étant limitée seulement par les revendications annexées.

La description se réfère aux dessins annexés sur lesquels :
- la figure 1 illustre schématiquement un aéronef connecté à un véhicule aéroportuaire équipé d'un dispositif de vidange et surveillance suivant un premier mode de réalisation de l'invention,
- la figure 2 illustre schématiquement en plus grand détail le véhicule aéroportuaire de la figure 1,
- la figure 3 illustre schématiquement l'organisation d'un aéroport équipé de plusieurs véhicules aéroportuaires comme celui des figures 1 et 2,
- la figure 4 illustre schématiquement un véhicule aéroportuaire équipé d'un dispositif de vidange et surveillance suivant un deuxième mode de réalisation de l'invention, et
- la figure 5 illustre schématiquement un aéronef dans une installation aéroportuaire fixe équipée d'un dispositif de vidange et surveillance suivant un exemple comparatif.

### Description détaillée de l'invention

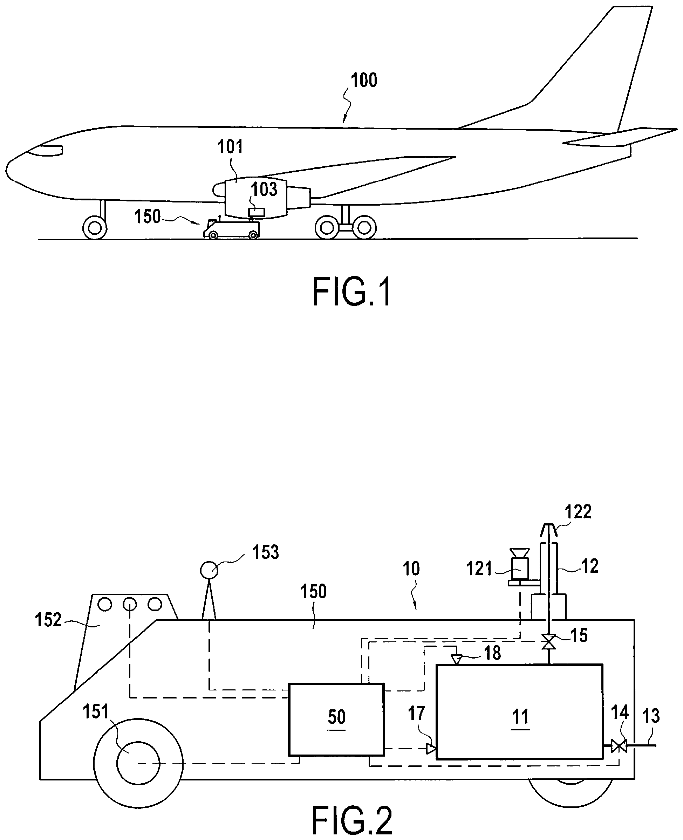

Comme illustré sur la figure 1, un aéronef 100 peut comprendre, par exemple dans une nacelle entourant le moteur 101, sous ce moteur 101, un réservoir embarqué 103 pour recevoir du fluide drainé du moteur 101, notamment en vol. Un dispositif de vidange et surveillance 10 suivant un premier mode de réalisation peut être installé à bord d'un véhicule aéroportuaire 150 pour son déplacement vers un aéronef 100 afin de procéder à la vidange d'un fluide drainé d'un moteur 101 de l'aéronef 100.

Comme illustré en plus grand détail sur la figure 2, ce dispositif de vidange et surveillance 10 peut comprendre un premier passage d'admission 12 avec une vanne 15, un premier réservoir 11 connecté à ce premier passage d'admission 12, un passage de vidange 13 du premier réservoir 11 avec une vanne 14, un premier ensemble capteur de qualité 17 et un premier capteur de niveau 18, installé dans le premier réservoir 11.

Le premier passage d'admission 12 peut être configuré pour recevoir, directement depuis l'aéronef 100, et plus précisément depuis un passage de vidange du réservoir embarqué 103, le fluide drainé du moteur 101. Pour cela, ce passage d'admission 12 peut être extensible et configuré pour établir une connexion hermétique avec le passage de vidange du réservoir embarqué 103. Ainsi, selon l'invention, le premier passage d'admission 12 est formé dans une perche articulée similaire à celles utilisées pour le ravitaillement en vol, possiblement équipée d'une caméra 121, ou d'un autre capteur, pour son guidage vers le passage de vidange du réservoir embarqué 103, et d'un connecteur 122, qui peut notamment être un connecteur mâle, pour la connexion hermétique avec le passage de vidange du réservoir embarqué 103. La perche peut être aussi articulée et télescopique.

La vanne 15 peut permettre l'obturation du passage d'admission 12 lorsqu'il n'est pas utilisé pour une vidange de fluide drainé d'un moteur d'aéronef, de manière à éviter la contamination du réservoir 11 avec d'autres substances.

Le premier réservoir 11 peut être configuré pour contenir, au moins temporairement, le fluide reçu depuis l'aéronef 100 et admis à travers le premier passage d'admission 12, de manière à permettre à l'ensemble capteur de qualité 17 de capter au moins un paramètre de qualité du fluide drainé du moteur 101, admis dans le dispositif de vidange et surveillance 10 à travers le premier passage d'admission 12, et contenu dans le premier réservoir 11, et au premier capteur de niveau 18 de capter le volume de fluide vidangé depuis l'aéronef 101 à travers le premier passage d'admission 12. L'ensemble capteur de qualité 17 peut notamment comprendre un capteur de viscosité du fluide drainé du moteur et/ou un ensemble capteur de polluants présents dans le fluide drainé du moteur. L'ensemble capteur de polluants peut notamment comprendre au moins un capteur de particules ferromagnétiques, un capteur de conductivité électrique, un capteur optique et/ou un capteur acoustique. Le capteur de viscosité et le capteur de particules ferromagnétiques peuvent être situés à l'extérieur du premier réservoir 11 et notamment en amont de celui-ci, comme par exemple dans le premier passage d'admission 12, en contact avec l'écoulement dynamique du fluide. Il est toutefois aussi envisageable de les installer dans le premier réservoir 11, ou dans un conduit annexe à celui-ci. Le capteur optique ou acoustique peut être configuré pour effectuer une mesure de transmission, absorption, réflexion et/ou réfraction du fluide contenu dans le réservoir embarqué, sur une ou plusieurs longueurs d'onde, notamment afin d'en caractériser la composition, décantation et/ou stratification de ce fluide dans le réservoir embarqué.

Le premier capteur de niveau 18 et le premier ensemble capteur de qualité 17 peuvent être connectés à l'unité de commande 50. L'unité de commande 50 peut être configurée pour déterminer, à partir des données captées par le premier capteur de niveau 18 et par le premier ensemble capteur de qualité 17, non seulement la quantité de fluide drainé par le moteur 101, mais aussi des caractéristiques physiques et/ou chimiques de celui-ci, et notamment sa teneur en polluants en suspension, y compris en eau et/ou en particules. L'unité de commande 50 peut par ailleurs comprendre une mémoire intégrée pour stocker ces données et être intégrée dans et/ou ou connectée à un système de pronostic et gestion de santé (en anglais : « Prognosis and health management » ou « PHM ») du moteur 101, embarqué à bord de l'aéronef 100 et/ou situé au sol, permettant d'effectuer un diagnostic du moteur 101 pour diriger la maintenance prévisionnelle de celui-ci en fonction de la quantité de fluide drainé du moteur 101 et des caractéristiques physiques et/ou chimiques de celui-ci, telles que déterminées par l'unité de commande 50 à partir des données capturées par le capteur de niveau 18 et par le premier ensemble capteur de qualité 17, seules ou en combinaison avec d'autres facteurs. Le système de pronostic et gestion de santé peut notamment être configuré pour diagnostiquer un défaut d'étanchéité au sein du moteur 101 et/ou une usure excessive de pièces mobiles du moteur 101, et éventuellement préconiser une opération de maintenance ou d'inspection, immédiate ou différée, et/ou autoriser le décollage de l'aéronef 100 en fonction de ce diagnostic.

Le passage de vidange 13 du dispositif de vidange et surveillance 10 peut être configuré pour permettre la vidange du réservoir 11 en ouvrant la vanne 14, de manière à libérer sa capacité pour procéder à la vidange et l'analyse du fluide drainé d'autres moteurs et/ou d'autres aéronefs. Comme illustré, les vannes 14 et 15 et/ou la perche formant le passage d'admission 12 peuvent aussi être connectés à une unité de commande 50, qui peut donc être configurée pour commander le guidage de la perche et sa connexion au passage de vidange du réservoir embarqué 103 et/ou l'ouverture et fermeture des vannes 14 et 15.

Comme illustré sur la figure 2, le véhicule aéroportuaire 150 peut comprendre aussi des capteurs 152, par exemple des capteurs optiques et/ou radar, un terminal de communication sans fil 153 et un dispositif de propulsion, direction et freinage 151 connectés à l'unité de commande 50 pour former un dispositif de pilotage autonome permettant de commander le déplacement du véhicule aéroportuaire 150, avec le dispositif de vidange et surveillance 10, à travers des installations aéroportuaires et jusqu'à l'aéronef.

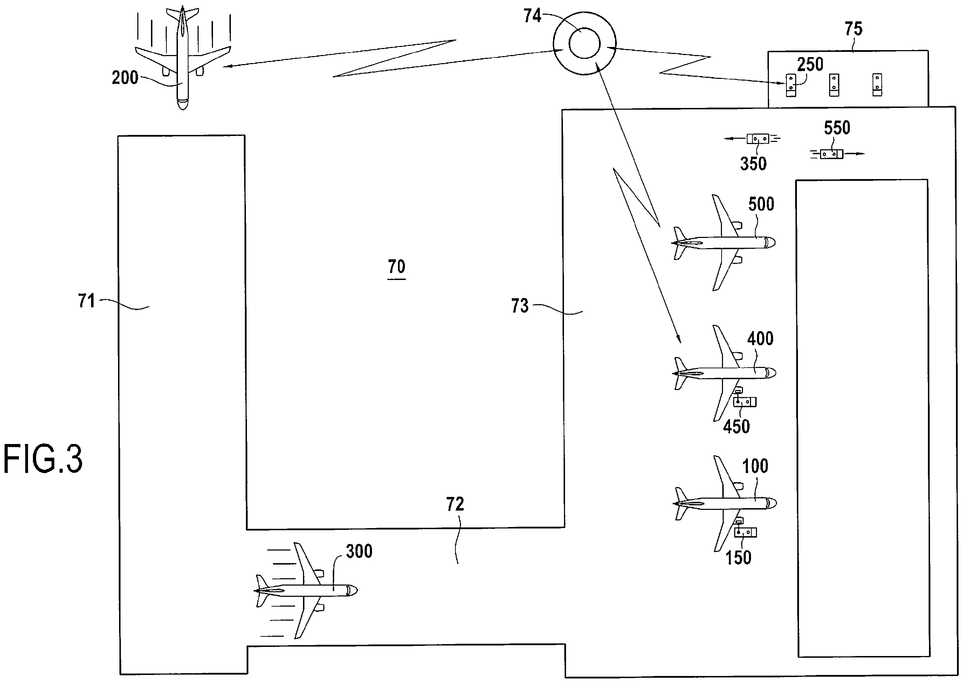

En fonctionnement, un véhicule aéroportuaire avec ces dispositifs de pilotage autonome et vidange et surveillance peut suivre un procédé tel que celui illustré sur la figure 3, dans laquelle les aéronefs 200, 300, 400 et 500 sont des aéronefs analogues à l'aéronef 100 et comportant des éléments équivalents, et les véhicules aéroportuaires 250, 450 et 550 sont des véhicules aéroportuaires analogues au véhicule aéroportuaire 150 et comportant aussi des éléments équivalents. Déjà en approche vers la piste d'atterrissage 71 de l'aéroport 70, l'aéronef 200 peut transmettre un signal à un centre de communication, par exemple à la tour de contrôle 74. Suite à ce signal, le véhicule aéroportuaire 250 correspondant, stationné dans une première zone de stationnement 75, peut être activé et recevoir instruction de se déplacer vers un emplacement, sur une deuxième zone de stationnement 73, désigné pour l'aéronef 200 en approche afin de procéder à sa vidange. Ainsi, pendant qu'un autre aéronef 300, ayant déjà atterri, se déplace sur une voie de circulation 72 vers son emplacement désigné sur la deuxième zone de stationnement 73, le véhicule aéroportuaire 350 correspondant peut déjà être en déplacement, piloté de manière autonome depuis la première zone de stationnement 75, pour le rencontrer à cet emplacement désigné.

A l'emplacement désigné pour l'aéronef 100, ou même quand celui-ci se trouve encore en mouvement sur la voie de circulation 72 vers la deuxième zone de circulation, le véhicule aéroportuaire 150 correspondant peut procéder à déployer automatiquement vers l'aéronef 100 la perche articulée formant le passage d'admission 12 de son dispositif de vidange et surveillance 10, éventuellement guidée par l'unité de commande 50 avec les images captées par la caméra 121, jusqu'à connecter le passage d'admission 12 de son dispositif de vidange et surveillance 10 au passage de vidange du réservoir embarqué 103 sur l'aéronef 100, comme déjà illustré sur la figure 1. La perche peut être aussi articulée et télescopique. Après cette connexion, qui peut être une connexion hermétique pour réduire le risque de pollution environnementale, l'unité de commande 50 peut commander l'ouverture de la vanne 15 pour procéder à l'admission, directement depuis l'aéronef 100 par le premier passage d'admission 12, du fluide drainé du moteur 101 et contenu dans le réservoir embarqué 103. Ce fluide est admis ainsi dans le dispositif de vidange et surveillance 10 à bord du véhicule aéroportuaire 150, où l'on peut procéder à la capture, à travers de l'ensemble capteur de qualité 17, d'au moins un paramètre de qualité du fluide, ainsi que de son volume à travers le capteur de niveau 18. Ces données peuvent être transmises par l'ensemble capteur de qualité 17 et par le capteur de niveau 18 à l'unité de commande 50 pour déterminer des caractéristiques physiques et/ou chimiques du fluide, et notamment sa teneur en polluants en suspension, y compris en eau et/ou en particules. Ces informations peuvent alors être transmises au système de pronostic et gestion de santé permettant de diriger la maintenance prévisionnelle de celui-ci.

Ainsi, par exemple, les données captées par le premier ensemble capteur de qualité 17 peuvent permettre d'identifier les proportions de lubrifiant, carburant et eau dans le fluide drainé par le moteur 101. L'unité de commande 50, en multipliant ces proportions par le volume total de fluide drainé, qui peut être déduit à partir des données captées par le premier capteur de niveau 18, peut obtenir les volumes totaux de lubrifiant, carburant et eau drainés par le moteur 101 vers le premier compartiment 11. Un défaut d'étanchéité des parties lubrifiées du moteur 101, qui peut notamment affecter la qualité de l'air prélevé au moteur 101 pour le système de pressurisation de la cabine de l'aéronef 100, peut être diagnostiqué par le système de pronostic et gestion de santé suite à la détection d'un volume excessif de lubrifiant dans le fluide drainé. La détection d'un volume excessif de carburant dans le fluide drainé, d'autre part, peut permettre au système de pronostic et gestion de santé de diagnostiquer un défaut d'étanchéité du circuit d'alimentation de carburant et/ou d'actionneurs utilisant le carburant comme fluide hydraulique. Les données captées par le premier ensemble capteur de qualité 17 peuvent aussi permettre d'identifier quantité et type de particules solides dans le fluide drainé par le moteur 101 vers le premier compartiment 11, distinguant notamment entre particules ferromagnétiques et particules riches en carbone. Le système de pronostic et gestion de santé peut ainsi diagnostiquer une usure excessive suite à la détection, à travers le premier ensemble capteur de qualité 17, d'une quantité excessive de particules ferromagnétiques dans ce fluide drainé, en particulier de particules ferromagnétiques de taille dépassant un seuil prédéterminé, tandis qu'une mauvaise combustion au sein d'une chambre de combustion du moteur 101 pourrait être diagnostiquée par le système de pronostic et gestion de santé suite à la détection d'une quantité excessive de particules riches en carbone dans ce même fluide drainé. Suite à ces diagnostics, le système de pronostic et gestion de santé peut aussi préconiser une opération de maintenance ou d'inspection, immédiate ou différée, et/ou autoriser le décollage de l'aéronef 100 en fonction de ce diagnostic.

Après que la vidange du réservoir embarqué ait été effectuée, comme dans le cas de l'aéronef 400 sur la figure 3, le véhicule aéroportuaire 450 correspondant peut procéder à retirer sa perche et transmettre un signal de fin de tâche, pour ensuite retourner, de manière aussi autonome, à la première zone de stationnement 75, comme le véhicule aéroportuaire 550 retournant de l'emplacement de l'aéronef 500 correspondant. De retour dans la première zone de stationnement 75, ou dans un autre endroit désigné pour la vidange du réservoir 11, l'unité de commande 50 peut commander l'ouverture de la vanne 14 du passage de vidange 13 du dispositif de vidange et surveillance 10, pour ainsi éliminer les fluides drainés du moteur 101 de manière contrôlée et écoresponsable.

Même si, dans le mode de réalisation illustré sur les figures 1 et 2, le dispositif de vidange et surveillance ne comporte qu'un seul passage d'admission, il est également envisageable qu'il en comporte une pluralité de passages d'admission distincts, permettant notamment l'admission séparée de fluides drainés de régions différentes du moteur 101 et contenus dans des compartiments séparés dans le réservoir embarqué 103, voire même dans une pluralité de réservoirs embarqués 103 distincts, avec des passages de vidange respectifs. Un ensemble capteur de qualité peut être associé à chaque passage d'admission, pour ainsi procéder séparément à l'analyse des fluides drainés de chaque région différente du moteur 101, afin d'assurer une surveillance et un diagnostic plus précis. En outre, le dispositif de vidange et surveillance peut aussi comprendre, en aval de l'un ou plusieurs ensembles capteurs de qualité, au moins un appareil de traitement du fluide drainé du moteur 101 pour faciliter l'élimination écoresponsable de ce fluide.

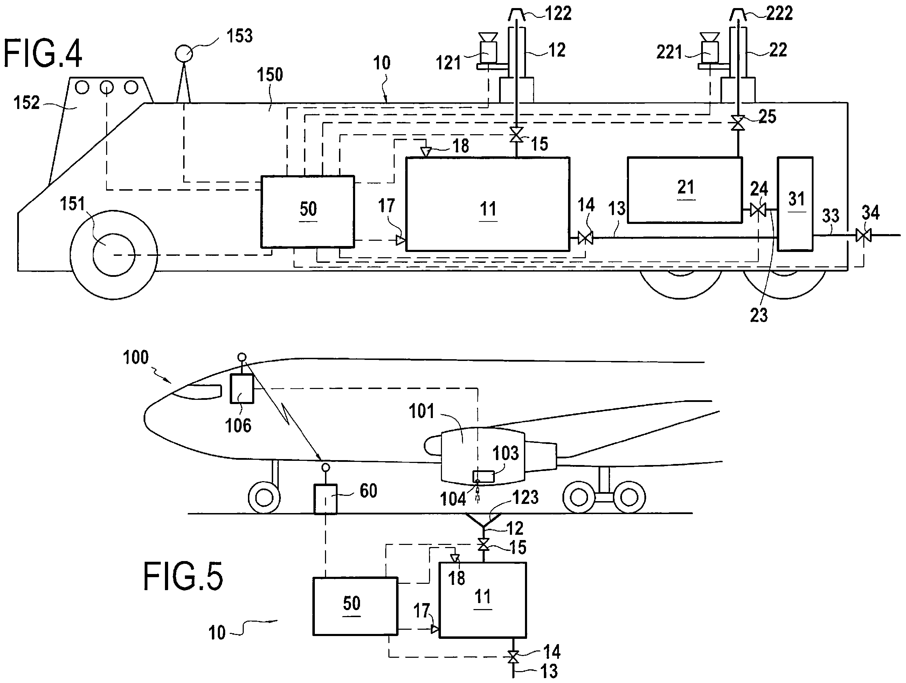

Ainsi, comme illustré sur la figure 4, le dispositif de vidange et surveillance 10 peut comprendre non seulement un premier passage d'admission 12 avec une vanne 15, un premier réservoir 11 connecté à ce premier passage d'admission 12, un passage de vidange 13 du premier réservoir 11 avec une vanne 14, un premier ensemble capteur de qualité 17 et un premier capteur de niveau 18, installé dans le premier réservoir 11, mais aussi un deuxième passage d'admission 22 avec une vanne 25, un deuxième réservoir 21 connecté à ce deuxième passage d'admission 22, un passage de vidange 23 du deuxième réservoir 21 avec une vanne 24, un deuxième ensemble capteur de qualité 27 et un deuxième capteur de niveau 28, installé dans le deuxième réservoir 21. Chacun des premiers et deuxième passages d'admission 12, 22, réservoirs 11, 21, ensembles capteurs de qualité 17, 27, capteurs de niveau 18, 28, et passages de vidange 15, 25 peuvent être respectivement équivalents au premier passage d'admission 12, réservoir 11, ensemble capteur de qualité 17, capteur de niveau 18, et passage de vidange 15 du dispositif de vidange et surveillance 10 illustré sur la figure 2, et fonctionner de manière analogue. Ainsi, selon l'invention, le premier passage d'admission 12 est formé dans une perche articulée similaire à celles utilisées pour le ravitaillement en vol, possiblement équipée d'une caméra 221, ou d'un autre capteur, connectée à l'unité de commande 50, pour son guidage vers un deuxième passage de vidange du réservoir embarqué 103, et d'un connecteur 222, qui peut notamment être un connecteur mâle, pour la connexion hermétique avec ce deuxième passage de vidange du réservoir embarqué 103. La perche du dispositif de vidange surveillance peut être aussi articulée et télescopique.

Ainsi, les premier et deuxième capteurs de niveau 18, 28 et les premier et deuxième ensembles capteurs de qualité 17, 27 peuvent être connectés à l'unité de commande 50, qui peut être configurée pour déterminer individuellement, à partir des données captées par ceux-ci, la quantité et les caractéristiques physiques et/ou chimiques du fluide drainé par le moteur 101, ayant été admis à travers chaque passage d'admission 12, 22. A part cela, l'unité de commande 50 peut être équivalente à celle du dispositif illustré sur la figure 2, et fonctionner de manière analogue.

Comme illustré aussi sur la figure 4, le dispositif de vidange et surveillance 10 peut aussi comprendre, en aval des passages de vidange 13, 23, un appareil de traitement 31, qui peut notamment être une centrifugeuse ou une cuve de décantation, avec son propre passage de vidange 33 équipé de sa propre vanne 34. Comme illustré, la vanne 34 peut aussi être connectée à l'unité de commande 50. Ainsi, entre la vidange des réservoirs 11, 21 par ouverture des vannes 14, 24 commandée par l'unité de commande 50, et celle de l'appareil de traitement 31 par ouverture de la vanne 34, cet appareil de traitement 31 peut procéder à un traitement du fluide provenant des réservoirs 11, 21, pour ainsi faciliter ensuite son élimination de manière écoresponsable à un endroit désigné pour la vidange de l'appareil de traitement 31. Les éléments restants du dispositif de vidange et surveillance 10 et du véhicule aéroportuaire 150 suivant la figure 4 sont équivalents à ceux de la figure 2 et reçoivent donc les mêmes repères.

Bien que dans les premier et deuxième modes de réalisation illustrés le dispositif de vidange et surveillance 10 soit intégré dans un véhicule aéroportuaire, il est également envisageable de l'intégrer dans une installation aéroportuaire fixe, comme illustré sur la figure 5, illustrant un exemple comparatif non couvert par les revendications. Dans cet exemple comparatif, comme illustré, le réservoir 11 peut être enfoui sous le tarmac de l'aéroport, et le passage d'admission 12 comprendre une cuvette 123 dans la surface du tarmac, disposée pour recevoir du fluide drainé du moteur 101 d'un aéronef 100 disposé à une station de vidange appropriée. Cette station de vidange peut, par exemple, être située dans une voie de circulation ou dans zone de stationnement d'aéronefs. Comme illustré, le dispositif de vidange et surveillance 10 peut comprendre une borne 60 en surface, connectée à l'unité de commande 50, et apte à établir une connexion sans fil avec un terminal embarqué 106 à bord de l'aéronef 100. Ce terminal embarqué 106 peut être connecté, à son tour, au passage de vidange 104 du réservoir embarqué 103, pour commander son ouverture et/ou fermeture. Les caractéristiques restantes du dispositif de vidange et surveillance 10 suivant cet exemple comparatif peuvent être analogues à celles de celui illustré sur la figure 2 et reçoivent les mêmes repères.

Ainsi, l'aéronef 100 peut être dirigé à cette station de vidange. Le terminal embarqué 106 peut établir une communication sans-fil avec la borne 60 et, quand il est établi que le passage de vidange 104 est situé directement au regard de la cuvette 123, le fluide drainé du moteur 101 et contenu dans le réservoir embarqué 103 peut ainsi être vidangé et s'écouler naturellement par gravité du réservoir embarqué 103 au réservoir 11 du dispositif de vidange et surveillance 10, en passant par le passage de vidange 104, et la cuvette 123 et le passage d'admission 12. Après que cette vidange soit complétée, le terminal embarqué 106 et l'unité de commande 50 peuvent commander, respectivement, la fermeture du passage de vidange 104 du réservoir embarqué 103 et celle du passage d'admission 12 du dispositif de vidange et surveillance 10, et l'aéronef 100 peut éventuellement repartir de cette station de vidange, tandis que l'on peut procéder, dans le réservoir 11, à la capture, à travers de l'ensemble capteur de qualité 17, d'au moins un paramètre de qualité du fluide, ainsi que de son volume à travers le capteur de niveau 18. Ces données peuvent être transmises par l'ensemble capteur de qualité 17 et par le capteur de niveau 18 à l'unité de commande 50 pour surveiller de cette manière l'état du moteur 101, éventuellement dans le cadre d'un système de pronostic et gestion de santé permettant de diriger la maintenance prévisionnelle de celui-ci. Après la capture de ces paramètres, l'unité de commande 50 peut encore procéder à commander l'ouverture de la vanne 14 du passage de vidange 13 du dispositif de vidange et surveillance 10, pour ainsi évacuer les fluides drainés du moteur 101 de manière contrôlée et écoresponsable.

Quoique la présente invention ait été décrite en se référant à des exemples de réalisation spécifiques, il est évident que des différentes modifications et changements peuvent être effectués sur ces exemples sans sortir de la portée générale de l'invention telle que définie par les revendications. En outre, des caractéristiques individuelles des différents modes de réalisation évoqués peuvent être combinées dans des modes de réalisation additionnels. Ainsi, par exemple, , l'installation aéroportuaire de l'exemple comparatif pourrait être équipée d'une perche extensible, plutôt que d'une cuvette. Par conséquent, la description et les dessins doivent être considérés dans un sens illustratif plutôt que restrictif.

## Revendications

1. Dispositif de vidange et surveillance (10) pour vidanger un réservoir embarqué (103) d'un fluide drainé d'un moteur (101) d'aéronef (100) et surveiller le fluide drainé du moteur (101) d'aéronef (100), le dispositif de vidange et surveillance (10) comprenant :
une perche articulée incorporant un premier passage d'admission (12) extensible apte à établir une connexion hermétique avec un passage de vidange disposé sur l'aéronef pour recevoir, directement depuis l'aéronef (100), le fluide drainé du moteur (101), et
un premier ensemble capteur de qualité (17) pour capter au moins un paramètre de qualité du fluide drainé du moteur (101) ayant été admis à travers le premier passage d'admission (12).

2. Dispositif de vidange et surveillance (10) suivant la revendication 1, dans lequel le premier ensemble capteur de qualité (17) comprend un capteur de viscosité pour capter une viscosité du fluide drainé du moteur (101).

3. Dispositif de vidange et surveillance (10) suivant l'une quelconque des revendications 1 ou 2, dans lequel le premier ensemble capteur de qualité (17) comprend un ensemble capteur de polluants pour capter une présence de polluants dans le fluide drainé du moteur (101).

4. Dispositif de vidange et surveillance (10) suivant l'une quelconque des revendications 1 à 3, comprenant en outre, en aval du premier ensemble capteur de qualité (17), au moins un appareil de traitement (31), pour le traitement du fluide drainé du moteur (101) ayant été admis à travers le premier passage d'admission (12).

5. Dispositif de vidange et surveillance (10) suivant l'une quelconque des revendications 1 à 4, comprenant en outre :
un deuxième passage d'admission (22) pour recevoir, directement depuis l'aéronef (100), du fluide drainé du moteur (101), et
un deuxième ensemble capteur de qualité (27) pour capter au moins un paramètre de qualité du fluide drainé du moteur (101) ayant été admis à travers le deuxième passage d'admission (22).

6. Dispositif de vidange et surveillance suivant l'une quelconque des revendications 1 à 5, dans laquelle la perche articulée est aussi télescopique.

7. Véhicule aéroportuaire (150) comprenant un dispositif de vidange et surveillance (10) suivant l'une quelconque des revendications 1 à 6.

8. Véhicule aéroportuaire (150) suivant la revendication 7, comprenant un dispositif de pilotage autonome.

9. Installation aéroportuaire fixe comprenant un dispositif de vidange et surveillance (10) suivant l'une quelconque des revendications 1 à 6.

10. Procédé de vidange et surveillance pour vidanger un réservoir embarqué (103) d'un fluide drainé d'un moteur (101) d'aéronef (100) et surveiller le fluide drainé du moteur (101) d'aéronef (100), le procédé de vidange et surveillance comprenant les étapes suivantes :
connexion hermétique, avec un passage de vidange sur l'aéronef (100), d'un premier passage d'admission (12) extensible incorporé dans une perche articulée d'un dispositif de vidange et surveillance (10),
admission, par le premier passage d'admission (12) du fluide drainé du moteur (101) d'aéronef (100), directement depuis l'aéronef (100), et
capture, à travers un premier ensemble capteur de qualité (17) dans le dispositif de vidange et surveillance (10), d'au moins un paramètre de qualité du fluide drainé du moteur (101) d'aéronef (100) ayant été admis à travers le premier passage d'admission (12).

11. Procédé de vidange et surveillance suivant la revendication 10, comprenant en outre une étape de déplacement du dispositif de vidange et surveillance (10), à bord d'un véhicule aéroportuaire (150), vers l'aéronef (100).

12. Procédé de vidange et surveillance suivant la revendication 11, comprenant un pilotage autonome du véhicule aéroportuaire pendant l'étape de déplacement.

13. Procédé de vidange et surveillance suivant l'une quelconque des revendications 10 à 12, dans lequel l'étape de connexion est effectuée de manière automatique.

14. Procédé de vidange et surveillance suivant l'une quelconque des revendications 10 à 13, dans lequel la perche articulée est aussi télescopique.

## Patentansprüche

1. Vorrichtung zur Entleerung und Überwachung (10) zum Entleeren eines Bordreservoirs (103) eines von einem Motor (101) eines Luftfahrzeugs (100) abgeführten Fluids und zum Überwachen des von dem Motor (101) des Luftfahrzeugs (100) abgeführten Fluids, wobei die Vorrichtung zur Entleerung und Überwachung (10) umfasst:
eine angelenkte Stange, die einen ersten ausfahrbaren Einlassdurchgang (12) beinhaltet, der dazu geeignet ist, eine hermetische Verbindung mit einem Entleerungsdurchgang zu bilden, der an dem Luftfahrzeug angeordnet ist, um direkt von dem Luftfahrzeug (100) das von dem Motor (101) abgeführte Fluid aufzunehmen, und
eine erste Anordnung von Qualitätssensoren (17) zum Erfassen zumindest eines Qualitätsparameters des von dem Motor (101) abgeführten Fluids, das durch den ersten Einlassdurchgang (12) eingelassen wurde.

2. Vorrichtung zur Entleerung und Überwachung (10) nach Anspruch 1, wobei die erste Anordnung von Qualitätssensoren (17) einen Viskositätssensor umfasst, um eine Viskosität des von dem Motor (101) abgeführten Fluids zu erfassen.

3. Vorrichtung zur Entleerung und Überwachung (10) nach einem der Ansprüche 1 oder 2, wobei die erste Anordnung von Qualitätssensoren (17) eine Anordnung von Verunreinigungssensoren umfasst, um ein Vorliegen von Verunreinigungen in dem von dem Motor (101) abgeführten Fluid zu erfassen.

4. Vorrichtung zur Entleerung und Überwachung (10) nach einem der Ansprüche 1 bis 3, umfassend ferner, der ersten Anordnung von Qualitätssensoren (17) nachgelagert, zumindest eine Behandlungseinrichtung (31) für die Behandlung des von dem Motor (101) abgeführten Fluids, das durch den ersten Einlassdurchgang (12) eingelassen wurde.

5. Vorrichtung zur Entleerung und Überwachung (10) nach einem der Ansprüche 1 bis 4, ferner umfassend:
einen zweiten Einlassdurchgang (22), um direkt von dem Luftfahrzeug (100) das Fluid, das von dem Motor (101) abgeführt ist, aufzunehmen, und
eine zweite Anordnung von Qualitätssensoren (27) zum Erfassen zumindest eines Qualitätsparameters des von dem Motor (101) abgeführten Fluids, das durch den zweiten Einlassdurchgang (22) eingelassen wurde.

6. Vorrichtung zur Entleerung und Überwachung nach einem der Ansprüche 1 bis 5, wobei die angelenkte Stange auch teleskopierbar ist.

7. Flughafenfahrzeug (150), umfassend eine Vorrichtung zur Entleerung und Überwachung (10) nach einem der Ansprüche 1 bis 6.

8. Flughafenfahrzeug (150) nach Anspruch 7, umfassend eine autonome Steuervorrichtung.

9. Stationäre Flughafenanlage, umfassend eine Vorrichtung zur Entleerung und Überwachung (10) nach einem der Ansprüche 1 bis 6.

10. Verfahren zur Entleerung und Überwachung zum Entleeren eines Bordreservoirs (103) eines von einem Motor (101) eines Luftfahrzeugs (100) abgeführten Fluids und zum Überwachen des von dem Motor (101) des Luftfahrzeugs (100) abgeführten Fluids, wobei das Verfahren zur Entleerung und Überwachung die folgenden Schritte umfasst:
hermetische Verbindung eines ersten ausfahrbaren Einlassdurchgangs (12), der in eine angelenkte Stange einer Vorrichtung zur Entleerung und Überwachung (10) inkorporiert ist, mit einem Entleerungsdurchgang an dem Luftfahrzeug (100),
Einlass des von dem Motor (101) des Luftfahrzeugs (100) abgeführten Fluids direkt von dem Luftfahrzeug (100) durch den ersten Einlassdurchgang (12), und
Erfassung zumindest eines Qualitätsparameters des von dem Motor (101) des Luftfahrzeugs (100) abgeführten Fluids, das durch den ersten Einlassdurchgang (12) eingelassen wurde, mittels einer ersten Anordnung von Qualitätssensoren (17) in der Vorrichtung zur Entleerung und Überwachung (10).

11. Verfahren zur Entleerung und Überwachung nach Anspruch 10, ferner umfassend einen Schritt der Bewegung der Vorrichtung zur Entleerung und Überwachung (10) an Bord eines Flughafenfahrzeugs (150) zu einem Luftfahrzeug (100) hin.

12. Verfahren zur Entleerung und Überwachung nach Anspruch 11, umfassend eine autonome Steuerung des Flughafenfahrzeugs während des Bewegungsschritts.

13. Verfahren zur Entleerung und Überwachung nach einem der Ansprüche 10 bis 12, wobei der Verbindungsschritt auf automatische Weise erfolgt.

14. Verfahren zur Entleerung und Überwachung nach einem der Ansprüche 10 bis 13, wobei die angelenkte Stange auch teleskopierbar ist.

## Claims

1. An emptying and monitoring device (10) for emptying an on-board reservoir (103) of a fluid drained from an aircraft (100) engine (101) and for monitoring the fluid drained from the aircraft (101) engine (101), the emptying and monitoring device (10) comprising:
an articulated pole incorporating an extensible first intake passage (12) configured to establish a leak-tight connection to an emptying passage disposed on the aircraft for receiving, directly from the aircraft (100), the fluid drained from the engine (101), and
a first quality sensor assembly (17) for detecting at least one quality parameter of the fluid drained from the engine (101) having been admitted through the first intake passage (12).

2. The emptying and monitoring device (10) according to claim 1, wherein the first quality sensor assembly (17) comprises a viscosity sensor for detecting a viscosity of the fluid drained from the engine (101).

3. The emptying and monitoring device (10) according to any one of claims 1 or 2, wherein the first quality sensor assembly (17) comprises a pollutant sensor assembly for detecting a presence of pollutants in the fluid drained from the engine (101).

4. The emptying and monitoring device (10) according to any one of claims 1 to 3, further comprising, downstream of the first quality sensor assembly (17), at least one treatment device (31) for treating the fluid drained from the engine (101) having been admitted through the first intake passage (12).

5. The emptying and monitoring device (10) according to any one of claims 1 to 4, further comprising:
a second intake passage (22) for receiving, directly from the aircraft (100), fluid drained from the engine (101), and
a second quality sensor assembly (27) for detecting at least one quality parameter of the fluid drained from the engine (101) having been admitted through the second intake passage (22).

6. The emptying and monitoring device (10) according to any one of claims 1 to 4, wherein the articulated pole is also telescoping.

7. An airport service vehicle (150) comprising an emptying and monitoring device (10) according to one of claims 1 to 6.

8. The airport service vehicle (150) according to claim 7, comprising an autonomous control device.

9. Fixed airport service installation comprising an emptying and monitoring device (10) according to any one of claims 1 to 6.

10. An emptying and monitoring method for emptying an on-board reservoir (103) of a fluid drained from an aircraft (100) engine (101) and monitoring the fluid drained from the aircraft (100) engine (101), the emptying and monitoring method comprising the following steps:
- connecting in a leak-tight manner, to an emptying passage disposed on the aircraft (100), an extensible first intake passage (12) incorporated in an articulated pole of an emptying and monitoring device (10),
- admitting, through the first intake passage (12) of fluid drained from the aircraft (100) engine (101) directly from the aircraft (100), and
- detecting, by means of a first quality sensor assembly (17) in the emptying and monitoring device (10), of at least one quality parameter of the fluid drained from the aircraft (100) engine (101) having been admitted through the first intake passage (12).

11. The emptying and monitoring method according to claim 10, further comprising a step of moving the emptying and monitoring device (10), on-board an airport service vehicle (150), to the aircraft (100).

12. The emptying and monitoring method according to claim 11, comprising autonomous control of the airport service vehicle during the movement step.

13. The emptying and monitoring method according to any one of claims 10 to 12, wherein the connecting step is accomplished automatically.

14. The emptying and monitoring method according to any one of steps 10 to 13, wherein the articulated pole is also telescoping.
